# EUROPEAN PATENT SPECIFICATION

(11) **EP 3 458 046 B1**
(45) Date of publication and mention of the grant of the patent: **13.01.2021**
(21) Application number: 17727386.9
(22) Date of filing: 19.05.2017
(51) Int. Cl.: A61K 31/23, A61K 31/202, A61K 31/185, A61K 31/198, A61K 31/20, A61K 31/205, A61K 31/355, A61K 33/14, A61K 45/06, A23K 50/40, A61K 9/00, A61P 9/00, A61P 43/00, A23K 10/00, A23K 20/158

(54) **MEDIUM-CHAIN TRIGLYCERIDES FOR THE TREATMENT OF DEGENERATIVE MITRAL VALVE DISEASE IN COMPANION ANIMALS**
MITTELKETTIGE TRIGLYCERIDE ZUR BEHANDLUNG DER DEGENERATIVEN MITRALKLAPPENINSUFFIZIENZ BEI HAUSTIEREN
TRIGLYCÉRIDES À CHAÎNE MOYENNE DESTINÉS AU TRAITEMENT DE L'INSUFFISANCE MITRALE DÉGÉNERATIVE CHEZ LES ANIMAUX DE COMPAGNIE

(30) Priority: 20.05.2016 US 201662339436 P
(43) Date of publication of application: 27.03.2019
(73) Proprietor: Société des Produits Nestlé S.A., 1800 Vevey (CH)
(72) Inventor: LI, Qinghong, Chesterfield, Missouri 63005 (US); LAFLAMME, Dorothy P., Floyd, Virginia 24091 (US); BOLER, Brittany Vester, St. Louis, Missouri 63116 (US); XU, Xui, Chesterfield, Missouri 63017 (US); BHATNAGAR, Sandeep, Ballwin, MO 63021 (US)
(74) Representative: Elkington and Fife LLP
(86) International application number: PCT/IB2017/052981
(87) International publication number: WO 2017/199223

(56) References cited:
- WO-A1-2014/090842
- WO-A1-2014/196957
- WO-A1-2014/209791
- WO-A2-2012/037328
- JEAN A. HALL ET AL: "Feeding Healthy Beagles Medium-Chain Triglycerides, Fish Oil, and Carnitine Offsets Age-Related Changes in Serum Fatty Acids and Carnitine Metabolites", PLOS ONE, vol. 7, no. 11, 7 November 2012 (2012-11-07), page e49510, XP055396819, DOI: 10.1371/journal.pone.0049510
- QINGHONG LI ET AL: "Veterinary Medicine and Multi-Omics Research for Future Nutrition Targets: Metabolomics and Transcriptomics of the Common Degenerative Mitral Valve Disease in Dogs", OMICS A JOURNAL OF INTEGRATIVE BIOLOGY, vol. 19, no. 8, 1 August 2015 (2015-08-01) , pages 461-470, XP055396836, NEW YORK, NY, US ISSN: 1536-2310, DOI: 10.1089/omi.2015.0057

## Description

### BACKGROUND

The present disclosure relates to compositions comprising medium-chain triglycerides for use in a method of preventing and/or treating degenerative mitral valve disease (DMVD) in a companion animal.

The mitral valve is located between the left atrium and left ventricle of the heart and is made of thin flaps of tissue, or valve leaflets, attached by long, tendon-like structures, the chordae tendineae, to the muscles of the left ventricle. The mitral valve helps regulate the flow of blood in and out of the heart and prevents a back flow from entering into the atrium. The valve leaflets open and close to regulate the flow of blood, but when the dog has degenerative mitral valve disease (DMVD) and the disease progresses, the valve leaflets begin to thicken, contract and lose flexibility.

When the mitral valve functions correctly, blood in the left ventricle is pumped to the body as the heart contracts. As the mitral valve degrades, it cannot close properly, and small amounts of blood leak back into the left atrium. Over time, the valve degrades until the heart can no longer compensate. Stress from the leak causes the heart to enlarge, eventually resulting in congestive heart failure. In severe cases, the chordae tendineae may rupture, damaging or causing the complete collapse of the mitral valve.

Cardiac disease is one of the most common health problems in dogs, and DMVD affects approximately 9% of all dogs, increasing with age such that the overall cumulative incidence is greater than 40%. Most owners do not learn their dog has DMVD until the condition reaches the advanced stage, when coughing, tiring after exercise, and a rapid respiratory rate can suggest that the dog has a health problem.

Surgical and medical procedures remain a viable option for treating DMVD. For example, medications such as diuretics can be given to help manage the fluid in the lungs and the arrhythmia, or irregular heartbeats, which often accompany DMVD. Nevertheless, these medications are not necessarily easily and safely used and furthermore can have side effects. Therefore, previous approaches have not satisfactorily addressed the problem of DMVD in dogs.

WO2014/209791 discloses nutritionally-balanced pet food compositions comprising MCTs, vitamin E and protein.

WO 2014/090842 discloses compositions comprising funny current (I_{f}) inhibitors for use in the treatment of heart disease in dogs, including the treatment of mitral valve insufficiency.

WO2012/037328 discloses compositions comprising medium-chain triglycerides, omega-3 oils (*e.g.* DHA and EPA) and vitamin E for use in the treatment of cardiovascular diseases in companion animals, including dogs.

WO2014/196957 discloses methods for diagnosing mitral valve insufficiency in dogs.

### SUMMARY

The present disclosure relates to compositions comprising medium-chain triglycerides for use in a method of preventing and/or treating degenerative mitral valve disease (DMVD) in a companion animal. More specifically, the present disclosure relates to compositions for a cardiac diet which comprises medium-chain triglycerides (MCT) and optionally one or more of omega-3 fatty acids, Vitamin E, magnesium, taurine, lysine, or sulfur-containing amino acids. The present disclosure also relates to methods using the compositions for cardiac protection and cardiac health for pets predisposed to DMVD or having DMVD.

To the best knowledge of the present inventors, currently there is no effective product for managing canine cardiac disease and more specifically, DMVD. For example, no existing cardiac diets are designed to address the alterations in energy metabolism that occur in dogs with DMVD.

The present inventors conducted a gene expression and metabolomics study on dogs with DMVD. The study indicated that long chain fatty acid beta-oxidation, branch chain fatty acid alpha oxidation, and ketolysis were compromised in these dogs, while glycolysis and glucose metabolism increased in dogs with DMVD compared with healthy controls. Several proteins and enzymes were compromised which are involved in transport of long chain fatty acids from outside the cell to the mitochondria inner matrix and activation of the long chain fatty acid in the cytoplasm. Without being bound by theory, the present inventors believe that MCT is able to enter the mitochondria matrix more quickly and easily with little assistance, and thus provide the critically needed energy of the ailing heart. Consequently, nutritional management can be very effective in prevention and early treatment of DMVD.

Accordingly, in a general embodiment, the present invention provides a composition comprising medium chain triglycerides for use in a method of preventing and/or treating degenerative mitral valve disease (DMVD) in a companion animal, wherein the medium chain triglyceride is a lipid in which three fatty acids are bound by ester linkages to a glycerol backbone, and at least two and preferably all three of the fatty acids are each between six and twelve carbons in length.

In an embodiment, the composition further comprises a component selected from the group consisting of an omega-3 fatty acid, vitamin E, magnesium, taurine, lysine, a sulfur-containing amino acid, and mixtures thereof. The omega-3 fatty acid can be selected from the group consisting of eicosapentaenoic acid (EPA), docosahexaenoic acid (DHA), and mixtures thereof. The sulfur-containing amino acid can be selected from the group consisting of methionine, cysteine, homocysteine, taurine and mixtures thereof.

In an embodiment, the composition can be a complete and nutritionally balanced pet food.

In an embodiment, the medium chain triglycerides can be 0.5 wt% to 60 wt% of the composition. In one aspect, the medium chain triglycerides can be from about 1 wt% to about 20 wt% of the composition. In other aspects, the medium chain triglycerides can be from about 1 wt% to about 15 wt%, from about 1 wt% to about 10 wt%, or from about 2 wt% to about 10 wt% of the composition.

In an embodiment, the composition further comprises a component selected from the group consisting of (i) carnitine, (ii) lysine and methionine, (iii) glutathione, and (iv) a mixture thereof.

In an embodiment, the composition has a characteristic selected from the group consisting of (i) a balanced level of magnesium/sodium/potassium, (ii) high protein, and (iii) combinations thereof. In one embodiment, a balanced level of magnesium/sodium/potassium can be provided by a ratio of potassium to sodium from 4:1 to 1:1 with the magnesium in an amount of 0.08 wt% to 0.25 wt% of the food composition.

In an embodiment, the composition can be administered to the companion animal daily for at least one week.

In an embodiment, the composition can be administered in an amount that provides 0.001 g to 50.0 g of the MCTs/ kg body weight of the companion animal per day. In one aspect, the amount can provide about 0.1 g to about 5 g of the MCTs/ kg body weight of the companion animal per day. In specific aspects, the amount can provide about 0.2 g to about 1.5 g, or even about 0.25 g to about 1 g, of the MCTs/ kg body weight of the companion animal per day.

In another embodiment, the method comprises orally administering a composition comprising a therapeutically effective amount of medium chain triglycerides to the companion animal. The medium chain triglycerides can be those discussed herein. The composition can further comprise components as discussed herein.

The composition can further comprise components as discussed herein.

In an embodiment, the companion animal is identified as being at risk of DMVD prior to the administering of the compositions to the companion animal. The identifying can comprise a step selected from the group consisting of (i) measuring a concentration of N-terminal pro B-type natriuretic peptide in the companion animal, (ii) detecting an enlarged heart of the companion animal, (iii) detecting a heart murmur in the companion animal, (iv) identifying a history of one or more of coughing, exercise intolerance, decreased appetite, difficulty breathing, or temporary loss of consciousness caused by a fall in blood pressure in the companion animal, (v) identifying the companion animal as a small breed, (vi) detecting mitral regurgitation, (vii) measuring changes in serotonin, transforming growth factor beta, or nitric oxide signaling pathways, (viii) measuring changes in energy metabolism including compromised long chain fatty acid beta-oxidation, compromised branched chain fatty acid alpha-oxidation, compromised ketolysis or increased glucose intake and glycolysis, and (ix) combinations thereof.

In one embodiment, the companion animal can be a canine.

An advantage of one or more embodiments provided by the present disclosure is heart-specific pet diet.

Yet another advantage of one or more embodiments provided by the present disclosure is treatment of DMVD.

Still another advantage of one or more embodiments provided by the present disclosure is the treatment of DMVD with minimal or no side effects.

Another advantage of one or more embodiments provided by the present disclosure is the treatment of DMVD using compositions easily and safely administered as a pet food or as an additive to pet food.

Additional features and advantages are described herein and will be apparent from, the following Detailed Description and the Figures.

### BRIEF DESCRIPTION OF THE FIGURES

**FIG. 1** is a graph of various cardiac measurements for dogs with cardiovascular disease in accordance with one embodiment of the present disclosure.
**FIG. 2** is a graph of various cardiac measurements for dogs with cardiovascular disease in accordance with one embodiment of the present disclosure.

### DETAILED DESCRIPTION

### Definitions

Some definitions are provided hereafter. Nevertheless, definitions may be located in the "Embodiments" section below, and the above header "Definitions" does not mean that such disclosures in the "Embodiments" section are not definitions.

As used in this disclosure and the appended claims, the singular forms "a," "an" and "the" include plural referents unless the context clearly dictates otherwise. Thus, for example, reference to "an ingredient" or "the ingredient" includes two or more ingredients. The term "and/or" used in the context of "X and/or Y" should be interpreted as "X," or "Y," or "X and Y." Where used herein, the term "example," particularly when followed by a listing of terms, is merely exemplary and illustrative, and should not be deemed to be exclusive or comprehensive.

As used herein, "about" is understood to refer to numbers in a range of numerals, for example the range of -10% to +10% of the referenced number, preferably within -5% to +5% of the referenced number, more preferably within -1% to +1% of the referenced number, most preferably within -0.1% to +0.1% of the referenced number. A range that is "between" two values includes those two values. Furthermore, all numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

All percentages expressed herein are by weight of the total weight of the composition unless expressed otherwise. When reference is made to the pH, values correspond to pH measured at 25°C with standard equipment.

The terms "food," "food product" and "food composition" mean a product or composition that is intended for ingestion by an animal and provides at least one nutrient to the animal. The term "pet food" means any food composition intended to be consumed by a dog.

The term "companion animal" means a dog or a cat. As used herein, the term "dog" and "canine" can be used interchangeably. In one embodiment, the companion animal can be a canine.

"Wet food" means a pet food having a moisture content from about 50% to about 90%, and in one aspect, from about 70% to about 90%. "Dry food" means a pet food having a moisture content less than about 20%, and in one aspect, less than about 15%, and in a specific aspect, less than about 10%. "Semi-moist food" means a pet food having a moisture content from about 20% to about 50%, and in one aspect, from about 25% to about 35%. "Kibbles" means pieces of dry or semi-moist pet food which can have a pellet shape or any other shape. Examples of kibbles include particulates; pellets; pieces of pet food, dehydrated meat, meat analog, vegetables, and combinations thereof; and pet snacks, such as meat or vegetable jerky, rawhide, and biscuits.

The compositions disclosed herein may lack any element that is not specifically disclosed herein. Thus, a disclosure of an embodiment using the term "comprising" includes a disclosure of embodiments "consisting essentially of' and "consisting of' the components identified. Similarly, the methods disclosed herein may lack any step that is not specifically disclosed herein. Thus, a disclosure of an embodiment using the term "comprising" includes a disclosure of embodiments "consisting essentially of' and "consisting of' the steps identified. Moreover, the description of some steps as "optional" does not imply that the other steps which are not explicitly described as optional are necessarily required.

Any embodiment disclosed herein can be combined with any other embodiment disclosed herein.

"Prevention" includes reduction of risk and/or severity of a condition or disorder. The terms "treatment," "treat" and "to alleviate" include both prophylactic or preventive treatment (that prevent and/or slow the development of a targeted pathologic condition or disorder) and curative, therapeutic or disease-modifying treatment, including therapeutic measures that cure, slow down, lessen symptoms of, and/or halt progression of a diagnosed pathologic condition or disorder; and treatment of patients at risk of contracting a disease or suspected to have contracted a disease, as well as patients who are ill or have been diagnosed as suffering from a disease or medical condition. The term does not necessarily imply that a subject is treated until total recovery. The terms "treatment" and "treat" also refer to the maintenance and/or promotion of health in an individual not suffering from a disease but who may be susceptible to the development of an unhealthy condition. The terms "treatment," "treat" and "to alleviate" are also intended to include the potentiation or otherwise enhancement of one or more primary prophylactic or therapeutic measure. The terms "treatment," "treat" and "to alleviate" are further intended to include the dietary management of a disease or condition or the dietary management for prophylaxis or prevention a disease or condition. A treatment can be patient- or doctor-related.

The relative terms "improved," "increased," "enhanced" and the like refer to the effects of the composition disclosed herein (a composition comprising a therapeutically effective amount of medium chain triglycerides or a prophylactic dose of medium chain triglycerides) relative to a composition having a lower amount or lacking medium chain triglycerides, but otherwise identical.

A "medium chain triglyceride" is a lipid in which three fatty acids are bound by ester linkages to a glycerol backbone, and at least two and preferably all three of the fatty acids are each between six and twelve carbons in length. The medium-chain fatty acids are caproic acid (comprising six carbon atoms or C6:0), caprylic acid (comprising eight carbon atoms or C8:0), capric acid (comprising ten carbon atoms or C10:0) and lauric acid (comprising twelve carbon atoms or C12:0). In one embodiment, the medium-chain fatty acids are mainly (e.g., at least 98%) in the form of triglycerides. A composition comprising "lipids consisting essentially of medium chain triglycerides" contains medium chain triglycerides as at least 20% of the lipids, in some embodiments at least 30% of the lipids, in other embodiments at least 40% of the lipids, and in some embodiments at least 50% of the lipids in the composition.

### Embodiments

An aspect of the present disclosure is a composition for use in a method of treating degenerative mitral valve disease (DMVD) in a companion animal having DMVD. Another aspect of the present disclosure is a composition for use in a method of preventing DMVD in a companion animal at risk thereof.

The methods comprise orally administering to the dog a composition comprising medium chain triglycerides and optionally one or more of omega-3 fatty acids, Vitamin E, magnesium, taurine, lysine, or sulfur-containing amino acids. The composition can be a pet food, such as a wet pet food, a semi-moist pet food, or a dry pet food, e.g., kibble.

Generally, the medium chain triglycerides can be 0.5 wt% to 60 wt% of the composition. In one aspect, the medium chain triglycerides can be from about 1 wt% to about 20 wt% of the composition. In other aspects, the medium chain triglycerides can be from about 1 wt% to about 15 wt%, from about 1 wt% to about 10 wt%, or from about 2 wt% to about 10 wt% of the composition. The medium chain triglycerides may be prepared by any known process, such as direct esterification, rearrangement, fractionation and/or transesterification. For example, the medium chain triglycerides may be prepared from a source of vegetable oil, such as coconut oil, through a rearrangement process. The chain length and distribution thereof may vary depending on the source oil. For example, MCTs containing 1-10% C6, 30-60% C8, 30-60% C10 and 1-10% C12 can be derived from palm oil and/or coconut oil; in some embodiments, at least a portion of the MCTs are provided by coconut oil, but in other embodiments the composition does not contain coconut oil. MCTs containing at least about 95% C8 can be made by semi-synthetic esterification of octanoic acid to glycerin; in some embodiments thereof, the remainder of the fatty acids are C6 and C10. Mixtures comprising MCTs with about 50% total C8 and/or about 50% total C10 are also useful herein.

Examples of suitable omega-3 fatty acids include eicosapentaenoic acid (EPA), docosahexaenoic acid (DHA), alpha-linolenic acid (ALA) and mixtures thereof. In one embodiment, the omega-3 fatty acids can range from 0.2 wt% to 3 wt% of the composition. In some embodiments, the omega-3 fatty acids are at least 0.2 wt%, at least about 1.0 wt%, or at least about 2.0 wt%.

Examples of suitable sulfur-containing amino acids include methionine, cysteine, homocysteine, taurine and mixtures thereof.

In the method of preventing DMVD in a companion animal at risk thereof, the method can comprise identifying the companion animal as being at risk of DMVD prior to the administering of the composition. Examples of characteristics that can identify a companion animal as being at risk of DMVD include an increased concentration of N-terminal pro B-type natriuretic peptide (NT-proBNP) and/or an enlarged heart relative to companion animals of the same gender, age and breed; the presence of a heart murmur, e.g., an apical systolic heart murmur; and a history of one or more of coughing, exercise intolerance, decreased appetite, difficulty breathing, or temporary loss of consciousness caused by a fall in blood pressure (e.g., over the previous month, for example over the previous six months). Additionally, other steps to identifying an at-risk companion animal include identifying the companion animal as a small breed, detecting mitral regurgitation, measuring changes in serotonin, transforming growth factor beta, or nitric oxide signaling pathways, and measuring changes in energy metabolism including compromised long chain fatty acid beta-oxidation, compromised branched chain fatty acid alpha-oxidation, compromised ketolysis or increased glucose intake and glycolysis. One or more steps can be combined to provide the identification.

In some embodiments, the composition can be administered to the companion animal for a time period of at least one week, at least one month, at least two, three, four, five or six months; and in some embodiments, for at least one year. During the time period, the composition can be administered to the dog at least one day per week, at least two days per week, at least three, four, five or six days per week; or even seven days per week. The composition can be administered in a single dose per day or in multiple separate doses per day. In an embodiment, the composition can be administered in an amount that provides 0.001 g to 50 g of the MCTs per kg body weight of the companion animal per day. In one aspect, 0.1 g to about 5 g of the MCTs per kg body weight of the companion animal can be administered per day.

In specific embodiments, the companion animal can be a canine.

In an embodiment, the composition further comprises (i) carnitine, (ii) lysine and methionine, (iii) an antioxidant such as glutathione, or (iv) mixtures thereof. The composition can be high in protein, for example at least 20 wt%, at least about 25 wt%, or even at least about 30 wt% of the composition. Additionally, the composition can have balanced amounts of magnesium, sodium and potassium; for example, the ratio of potassium to sodium can be 4:1 to 1:1, in one aspect, about 4:1 to about 2:1, with the magnesium in an amount of 0.08 wt% to 0.25 wt%, and in one aspect, from about 0.10 wt% to about 0.15 wt%. At least a portion of the magnesium, sodium and potassium can be provided as isolated compounds (e.g., salts). Alternatively or additionally, at least a portion of the magnesium, sodium and potassium can be provided by one or more foodstuffs. For example, magnesium can be provided by wheat bran, whole grains, leafy green vegetables, meat, beans and bananas; and potassium and sodium can be provided by meats, fish, whole grains, yogurt, bananas, sweet potatoes, squash, beans and tomatoes.

The pet food compositions disclosed herein can be any food formulated for consumption by a pet such as a dog. In an embodiment, the pet food composition provides complete nutrition as defined by the Association of American Feed Control Officials (AAFCO) and which depends on the type of animal for which the composition is intended (e.g., a dog).

The pet food composition can comprise meat, such as emulsified meat. Examples of suitable meat include poultry, beef, pork, lamb and fish, especially those types of meats suitable for pets. The meat can include any additional parts of an animal including offal. Some or all of the meat can be provided as one or more meat meals, namely meat that has been dried and ground to form substantially uniform-sized particles and as defined by AAFCO. Additionally or alternatively, vegetable protein can be used, such as pea protein, corn protein (e.g., ground corn or corn gluten), wheat protein (e.g., ground wheat or wheat gluten), soy protein (e.g., soybean meal, soy concentrate, or soy isolate), rice protein (e.g., ground rice or rice gluten) and the like.

The pet food compositions disclosed herein can comprise one or more of a vegetable oil, a flavorant, a colorant or water. Examples of suitable vegetable oils include soybean oil, corn oil, cottonseed oil, sunflower oil, canola oil, peanut oil, safflower oil and the like. In some embodiments, the lipids in the composition can consist of the MCTs and one or more of any vegetable oil, any fish oil, the lipid from any meat, and any omega-3 fatty acids.

Examples of suitable flavorants include yeast, tallow, rendered animal meals (e.g., poultry, beef, lamb, pork), flavor extracts or blends (e.g., grilled beef), animal digests, and the like. Examples of suitable colorants include FD&C colors, such as blue no. 1, blue no. 2, green no. 3, red no. 3, red no. 40, yellow no. 5, and yellow no. 6; natural colors, such as caramel coloring, annatto, chlorophyllin, cochineal, betanin, turmeric, saffron, paprika, lycopene, elderberry juice, pandan, and butterfly pea; titanium dioxide; and any suitable food colorant known to the skilled artisan.

The pet food compositions disclosed herein can optionally include additional ingredients, such as starches, humectants, oral care ingredients, preservatives, amino acids, fibers, prebiotics, sugars, animal oils, aromas, other oils additionally or alternatively to vegetable oil, salts, vitamins, minerals, probiotic microorganisms, bioactive molecules or combinations thereof.

Examples of suitable starches include a grain such as corn, rice, wheat, barley, oats, potatoes, peas, beans, cassava, and mixtures of these grains, and can be included at least partially in any flour. Examples of suitable humectants include salt, sugars, propylene glycol and polyhydric glycols such as glycerin and sorbitol. Examples of suitable oral care ingredients include alfalfa nutrient concentrate containing chlorophyll, sodium bicarbonate, phosphates (e.g., tricalcium phosphate, acid pyrophosphates, tetrasodium pyrophosphate, metaphosphates, and orthophosphates), peppermint, cloves, parsley, and ginger . Examples of suitable preservatives include potassium sorbate, sorbic acid, sodium methyl para-hydroxybenzoate, calcium propionate, propionic acid, and combinations thereof.

Specific amounts for each additional ingredient in the pet food compositions disclosed herein will depend on a variety of factors such as the ingredient included in the first edible material and any second edible material; the species of animal; the animal's age, body weight, general health, sex, and diet; the animal's consumption rate; and/or the purpose for which the food product is administered to the animal. Therefore, the components and their amounts may vary widely.

The pet food composition can be made by a method comprising adding MCTs to at least one other comestible ingredient, the MCTs being added in an amount effective to prevent and/or treat DMVD. For example, the MCTs can be added such that a single serving of the pet food comprises an amount of the MCTs effective to prevent and/or treat DMVD. Optionally one or more of omega-3 fatty acids, Vitamin E, magnesium, taurine, lysine, or sulfur-containing amino acids can be included in the pet food.

### EXAMPLE

By way of example, the following study is illustrative of compositions and methods using MCTs for the treatment of DMVD in dogs, in one or more embodiments provided by the present disclosure.

### Example 1 - Cardiac Health Blend Study for Canines with DMVD

19 healthy dogs and 21 with cardiac murmurs were selected for a 6-month study. All dogs were examined by a board-certified veterinary cardiologist using echocardiography and were confirmed as either healthy or having early stage degenerative mitral valve disease (DMVD). Dogs in each group were randomized by age, gender, breed, body weight, and murmur grades into two dietary treatment groups. The control diet provides complete and balanced nutrition manufactured by the Nestle Purina PetCare Company to meet or exceed the requirements as defined by the Association of American Feed Control Officials (AAFCO). The test diet contained all the ingredients in the control diet with the following additional ingredients: medium chain triglycerides (MCTs) in an amount of 5.000 wt%, omega-3 fatty acids in an amount of 0.7489 wt%, lysine in an amount of 2.3000 wt%, methionine and cysteine in an amount of 2.1000 wt%, magnesium sulfate in an amount of 0.1500 wt%, vitamin E in an amount of 0.9206 IU/g, and taurine in an amount of 0.2162 wt%. In the DMVD group, there were 10 dogs in the control diet group and 11 in the test diet group, while in the healthy group, there were 9 in the control diet group and 10 in the test diet group.

Dogs were individually fed to maintain their body weights. Energy intake for maintenance (MER) was estimated using the equation: MER = 139 ^{∗} BW^{0.67} (kilocalories), where BW is the body weight of the dog in kilograms. Dogs were weighed weekly and their amount of food offered was increased or decreased by 5% if their BW decreased or increased more than 5% over their initial BW, respectively. Echocardiographic parameters were measured by the cardiologist at the baseline, 3-month, and 6-month post treatment. Dogs received routine healthcare by the staff veterinarians. One DMVD dog of the control diet group was removed after 3 months due to health issues.

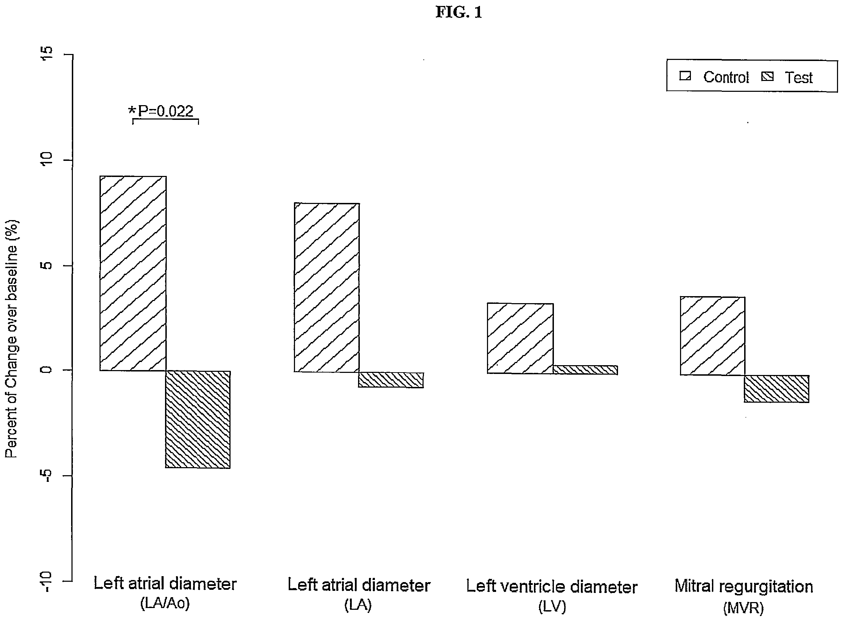

As shown in FIG. 1, echocardiograms were performed at baseline, 3 months, and 6 months post treatment. Changes between 3 months and baseline in the 4 cardiac measurements, left atrial to aortic root ratio (LA/Ao), left atrial diameter (LA), and left ventricular diameter (LV), and mitral regurgitation rate (MVR), were calculated. Mann-Whitney U test was performed to compare the differences between dogs fed control diet and dogs fed MCT diet and P values were obtained. While control diet-fed dogs had shown declines in all 4 measurements, improvements were observed in the test diet-fed dogs. Statistical significance (P<0.05) was denoted using an asterisk.

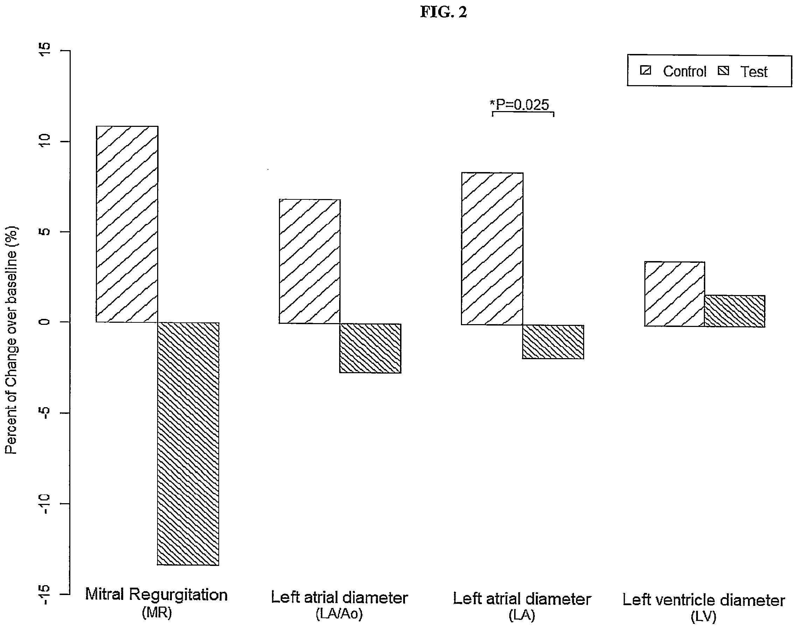

As shown in FIG. 2, echocardiograms were performed at baseline, 3 months, and 6 months post treatment. Changes between 6 months and baseline in the 4 cardiac measurements, mitral regurgitation grade (MR), left atrial to aortic root ratio (LA/Ao), left atrial diameter (LA), and left ventricular diameter (LV), were calculated. Mann-Whitney U test was performed to compare the differences between dogs fed control diet and dogs fed MCT diet and P values were obtained. While control diet-fed dogs had shown declines in all 4 measurements, improvements were observed in the test diet-fed dogs. Statistical significance (P<0.05) was denoted using an asterisk.

The data in FIGs. 1-2 demonstrate that while the control diet-fed DMVD dogs showed declines in key echocardiographic parameters over the course of the study, the test diet-fed DMVD dogs showed improvements. No significant difference was observed between the dietary groups in healthy dogs.

### Example 2 - Cardiac Health Blend Study for Canines with DMVD

Seven Beagles and 1 Miniature Schnauzer, all of which were examined and classified with degenerative mitral valve disease (DMVD) by a board-certified veterinary cardiologist using echocardiography, were selected for the study. Dogs were randomly assigned into two dietary treatment groups. The control diet provides complete and balanced nutrition manufactured by the Nestle Purina PetCare Company to meet or exceed the requirements as defined by the Association of American Feed Control Officials (AAFCO). The test diet contained the same ingredients as in the control diet except an additional 7% medium chain triglycerides (MCTs). The diets are isocaloric and isonitrogenous. The diets are shown in table 1.

**Table 1**

| Ingredients/Nutrients | Control Diet | Test Diet |
|---|---|---|
| Medium chain triglycerides (MCT), % | 0.0 | 7.0 |
| Omega-3 fatty acids, % | 0.8 | 0.8 |
| Lysine, % | 2.3 | 2.3 |
| Methionine + cysteine, % | 2.1 | 2.1 |
| Magnesium, % | 0.15 | 0.15 |
| Vitamin E, IU/g | 0.9 | 0.89 |
| Taurine, % | 0.21 | 0.21 |
| Carnitine, mg/kg | 750 | 750 |
| Sodium | 0.22 | 0.22 |
| Protein | 29.87 | 29.91 |
| Fat | 18.32 | 18.44 |
| Carbohydrates | 36.24 | 36.29 |

Dogs were individually fed to maintain their body weights. Energy intake for maintenance (MER) was estimated using the equation: MER = 139 ^{∗} BW^{0.67} (kilocalories), where BW is the body weight of the dog in kilograms. Dogs were weighed weekly and their amount of food offered was increased or decreased by 5% if their BW decreased or increased more than 5% over their initial BW, respectively. Echocardiogram variables were measured at the baseline and 3 months by the same cardiologist, who was blinded with diet assignments. Dogs received routine healthcare by the staff veterinarians. The results are shown in Tables 2 and 3.

**Table 2**

| | | | Murmur Grade | | Mitral Regurgitation Grade | | Mitral Regurgitation Velocity | |
|---|---|---|---|---|---|---|---|---|
| Breed | Sex | Diet | 3 month | Baseline | 3 month | Baseline | 3 month | Baseline |
| Beagle | M | Control | 4 | 4 | 3+ | 3+ | 6.01 | 5.08 |
| Beagle | F | Control | 2 | 2 | 2+ | 2+ | 5.74 | 5.37 |
| Miniature Schnauzer | M | Control | 2 | 2 | 2+ | 2+ | 5.98 | 5.94 |
| Beagle | F | Control | 3 | 3 | 2+ | 2+ | 6.28 | 5.41 |
| Beagle | F | Test | 2 | 3 | 2+ | 3+ | 5.54 | 5.85 |
| Beagle | F | Test | 1 | 1 | 1+ | 1+ | n/a | n/a |
| Beagle | M | Test | 3 | 3 | 3+ | 3+ | 5.73 | 5.91 |
| Beagle | M | Test | 1 | 2 | trace | 2+ | n/a | 6.52 |

**Table 3**

| | | | LA/Ao | | LVDd mmode (cm) | |
|---|---|---|---|---|---|---|
| Breed | Sex | Diet | 3 month | Baseline | 3 month | Baseline |
| Beagle | M | Control | 1.3 | 1.53 | 3.67 | 3.59 |
| Beagle | F | Control | 1.15 | 1.19 | 3.04 | 2.92 |
| Miniature Schnauzer | M | Control | 1.05 | 1.24 | 3.06 | 2.94 |
| Beagle | F | Control | 1.22 | 1.3 | 3.16 | 3.04 |
| Beagle | F | Test | 1.12 | 1.37 | 4.05 | 3.66 |
| Beagle | F | Test | 0.86 | 0.91 | 2.95 | 3 |
| Beagle | M | Test | 0.99 | 1.38 | 3.48 | 3.58 |
| Beagle | M | Test | 0.99 | 1.17 | 2.98 | 3.12 |

As shown in Tables 1 and 2, canines on the test diet generally had better murmur grades, mitral regurgitation grades, mitral regurgitation velocity, LA/ao, and LVDs mmode.

## Claims

1. A composition comprising medium chain triglycerides for use in a method of preventing and/or treating degenerative mitral valve disease (DMVD) in a companion animal, wherein the medium chain triglyceride is a lipid in which three fatty acids are bound by ester linkages to a glycerol backbone, and at least two of the fatty acids are each between six and twelve carbons in length.

2. The composition for use according to Claim 1, wherein the composition further comprises a component selected from the group consisting of an omega-3 fatty acid, Vitamin E, magnesium, taurine, lysine, a sulfur-containing amino acid, and mixtures thereof.

3. The composition for use according to Claim 2, wherein the omega-3 fatty acid is selected from the group consisting of eicosapentaenoic acid (EPA), docosahexaenoic acid (DHA), and mixtures thereof.

4. The composition for use according to Claim 2, wherein the sulfur-containing amino acid is selected from the group consisting of methionine, cysteine, homocysteine, taurine and mixtures thereof.

5. The composition for use according to Claim 1, wherein the composition is a complete and nutritionally balanced pet food.

6. The composition for use according to Claim 1, wherein the medium chain triglycerides are 0.5 wt% to 60 wt% of the composition.

7. The composition for use according to Claim 1, wherein the medium chain triglycerides include a medium-chain fatty acid selected from the group consisting of caprylic acid, capric acid, and a mixture thereof.

8. The composition for use according to Claim 1, wherein the composition further comprises a component selected from the group consisting of carnitine, lysine and methionine, glutathione, and a mixture thereof.

9. The composition for use according to Claim 1, wherein the composition has a characteristic selected from the group consisting of (i) ratio of potassium to sodium from 4:1 to 1:1, (ii) magnesium in an amount from 0.08 wt% to 0.25 wt%, (iii) protein in an amount of at least 20 wt%, and (iv) combinations thereof.

10. The composition for use according to Claim 1, wherein the composition is administered to the companion animal daily for at least one week.

11. The composition for use according to Claim 1, wherein the composition is administered in an amount that provides 0.001 g to 50.0 g of the MCTs per kg body weight of the companion animal per day.

12. The composition for use according to Claim 1, wherein the companion animal is a canine.

13. The composition for use according to Claim 1, wherein the companion animal is identified as being at risk of DMVD prior to the administering of the composition to the companion animal.

## Patentansprüche

1. Zusammensetzung, umfassend mittelkettige Triglyceride zur Verwendung in einem Verfahren zum Vorbeugen und/oder Behandeln degenerativer Mitralklappeninsuffizienz (DMVD) bei einem Haustier, wobei das mittelkettige Triglycerid ein Lipid ist, in dem drei Fettsäuren durch Esterbindungen an ein Glyceringerüst gebunden sind und mindestens zwei der Fettsäuren jeweils zwischen sechs und zwölf Kohlenstoffatome lang sind.

2. Zusammensetzung zur Verwendung nach Anspruch 1, wobei die Zusammensetzung ferner einen Bestandteil umfasst, der ausgewählt ist aus der Gruppe bestehend aus einer Omega-3-Fettsäure, Vitamin E, Magnesium, Taurin, Lysin, einer schwefelhaltigen Aminosäure und Mischungen davon.

3. Zusammensetzung zur Verwendung nach Anspruch 2, wobei die Omega-3-Fettsäure ausgewählt ist aus der Gruppe bestehend aus Eicosapentaensäure (EPA), Docosahexaensäure (DHA) und Mischungen davon.

4. Zusammensetzung zur Verwendung nach Anspruch 2, wobei die schwefelhaltige Aminosäure ausgewählt ist aus der Gruppe bestehend aus Methionin, Cystein, Homocystein, Taurin und Mischungen davon.

5. Zusammensetzung zur Verwendung nach Anspruch 1, wobei die Zusammensetzung ein vollwertiges und ernährungsphysiologisch ausgewogenes Heimtierfutter ist.

6. Zusammensetzung zur Verwendung nach Anspruch 1, wobei die mittelkettigen Triglyceride 0,5 Gew.-% bis 60 Gew.-% der Zusammensetzung ausmachen.

7. Zusammensetzung zur Verwendung nach Anspruch 1, wobei die mittelkettigen Triglyceride eine mittelkettige Fettsäure einschließen, die ausgewählt ist aus der Gruppe bestehend aus Caprylsäure, Caprinsäure und einer Mischung davon.

8. Zusammensetzung zur Verwendung nach Anspruch 1, wobei die Zusammensetzung ferner einen Bestandteil umfasst, der ausgewählt ist aus der Gruppe bestehend aus Carnitin, Lysin und Methionin, Glutathion und einer Mischung davon.

9. Zusammensetzung zur Verwendung nach Anspruch 1, wobei die Zusammensetzung eine Eigenschaft aufweist, die ausgewählt ist aus der Gruppe bestehend aus (i) einem Verhältnis von Kalium zu Natrium von 4 : 1 bis 1 : 1, (ii) Magnesium in einer Menge von zu 0,08 Gew.-% bis 0,25 Gew.-%, (iii) Protein in einer Menge von mindestens zu 20 Gew.-% und (iv) Kombinationen davon.

10. Zusammensetzung zur Verwendung nach Anspruch 1, wobei die Zusammensetzung dem Haustier täglich für mindestens eine Woche verabreicht wird.

11. Zusammensetzung zur Verwendung nach Anspruch 1, wobei die Zusammensetzung in einer Menge verabreicht wird, die 0,001 g bis 50,0 g der MCTs pro kg Körpergewicht des Haustiers pro Tag bereitstellt.

12. Zusammensetzung zur Verwendung nach Anspruch 1, wobei das Haustier ein Hund ist.

13. Zusammensetzung zur Verwendung nach Anspruch 1, wobei das Haustier vor der Verabreichung der Zusammensetzung an das Haustier als DMVDgefährdet identifiziert wird.

## Revendications

1. Composition comprenant des triglycérides à chaîne moyenne pour utilisation dans un procédé de prévention et/ou de traitement d'une maladie dégénérative de la valvule mitrale (DMVD) chez un animal de compagnie, dans laquelle le triglycéride à chaîne moyenne est un lipide dans lequel trois acides gras sont liés par des liaisons ester à un squelette glycérol, et au moins deux des acides gras ont chacun une longueur comprise entre six et douze carbones.

2. Composition pour utilisation selon la revendication 1, dans laquelle la composition comprend en outre un composant choisi dans le groupe constitué d'acide gras oméga-3, Vitamine E, magnésium, taurine, lysine, un acide aminé contenant du soufre, et des mélanges de ceux-ci.

3. Composition pour utilisation selon la revendication 2, dans laquelle l'acide gras oméga-3 est choisi dans le groupe constitué d'acide eicosapentaénoïque (EPA), acide docosahexaénoïque (DHA), et des mélanges de ceux-ci.

4. Composition pour utilisation selon la revendication 2, dans laquelle l'acide aminé contenant du soufre est choisi dans le groupe constitué de méthionine, cystéine, homocystéine, taurine et des mélanges de celles-ci.

5. Composition pour utilisation selon la revendication 1, dans laquelle la composition est un aliment complet et nutritionnellement équilibré pour animal domestique.

6. Composition pour utilisation selon la revendication 1, dans laquelle les triglycérides à chaîne moyenne représentent 0,5 % en poids à 60 % en poids de la composition.

7. Composition pour utilisation selon la revendication 1, dans laquelle les triglycérides à chaîne moyenne incluent un acide gras à chaîne moyenne choisi dans le groupe constitué d'acide caprylique, acide caprique, et un mélange de ceux-ci.

8. Composition pour utilisation selon la revendication 1, dans laquelle la composition comprend en outre un composant choisi dans le groupe constitué de carnitine, lysine et méthionine, glutathion, et un mélange de ceux-ci.

9. Composition pour utilisation selon la revendication 1, dans laquelle la composition a une caractéristique choisie dans le groupe constitué de (i) rapport du potassium au sodium allant de 4:1 à 1:1, (ii) magnésium en une quantité allant de 0,08 % en poids à 0,25 % en poids, (iii) protéines en une quantité d'au moins 20 % en poids, et (iv) des combinaisons de celles-ci.

10. Composition pour utilisation selon la revendication 1, dans laquelle la composition est administrée à l'animal de compagnie quotidiennement pendant au moins une semaine.

11. Composition pour utilisation selon la revendication 1, dans laquelle la composition est administrée en une quantité qui fournit 0,001 g à 50,0 g des TCM par kg de poids corporel de l'animal de compagnie par jour.

12. Composition pour utilisation selon la revendication 1, dans laquelle l'animal de compagnie est un canidé.

13. Composition pour utilisation selon la revendication 1, dans laquelle l'animal de compagnie est identifié comme étant à risque de DMVD avant l'administration de la composition à l'animal de compagnie.
